Europäisches Patentamt

European Patent Office

Office européen des brevets

(19)

(11) Publication number: **0 111 551**
**B1**

(12) **EUROPEAN PATENT SPECIFICATION**

(45) Date of publication of patent specification: **06.05.87**

(21) Application number: **83902218.3**

(22) Date of filing: **08.06.83**

(88) International application number:
**PCT/US83/00914**

(87) International publication number:
**WO 84/00003 05.01.84 Gazette 84/01**

(51) Int. Cl.⁴: **A 61 B 5/00**

(54) PROCESS AND APPARATUS FOR MEASURING BLOOD VISCOSITY DIRECTLY AND RAPIDLY.

(30) Priority: **14.06.82 US 388402**

(43) Date of publication of application:
**27.06.84 Bulletin 84/26**

(45) Publication of the grant of the patent:
**06.05.87 Bulletin 87/19**

(84) Designated Contracting States:
**DE FR GB**

(56) References cited:
US-A-3 071 961
US-A-3 194 057
US-A-3 720 097
US-A-3 908 441
US-A-4 207 870
US-A-4 367 754

No relevant documents have been disclosed.
Physics Education, March 1975, C. BOWLT, A simple capillary viscometer, vol. 10, no. 2, page 102-013

(73) Proprietor: **MERRILL, Edward W.**
**90 Somerset Street**
**Belmont, MA 02178 (US)**

(72) Inventor: **MERRILL, Edward W.**
**90 Somerset Street**
**Belmont, MA 02178 (US)**

(74) Representative: **Patentanwälte Zellentin**
**Zweibrückenstrasse 15**
**D-8000 München 2 (DE)**

(56) References cited:
The Review of Scientific Instruments, October 1954, SWANK et al, apparatus for Measuring Relative Blood Viscosity, vol. 25, no. 10, page 1020-1022

Med. and Biol. Engineering, September 1976, vol. 14, no. 5, WALKER et al., Measurement of Blood Viscosity using a conicylindrical viscometer, page 10551557

Jour. of Chemical Education, April 1971, vol. 48, no. 4, SORRELL, Liquid viscosity Measurement using a Buret, pp. 252-254
Polymer Sci., 1974, vol. 16, no. 1, Shvestka, Methods of Investigation Automatic capillary Viscometer, p. 222-227

## Description

Background of the invention

This invention relates to an apparatus for measuring the resistance to flow of a patient's blood under conditions approximating the microcirculatory vessels. Resistance to flow of blood is measured as an apparent viscosity, the flow taking place through a porous bed. The apparent viscosity of blood decreases from indefinitely large values near zero flow rates to an asymptotic value of the order of 0,003 to 0,005 N.S/m² (3 to 5 centipoise) above wall shear stresses of the order of 0,5 N/m² (5 dyn/cm²).

It is important to provide a screening test for large patient populations to determine a patient's blood shear-rate dependent viscosity in order to determine whether further analysis is required to measure the factors in the blood that affect blood viscosity. The non-Newtonian behavior of blood viscosity is determined by hematocrit (red cell volume percent) and macromolecular concentrations, primarily fibrinogen which in turn determines the ease of blood flow through microvasculature of the body and this varies widely from patient to patient. When blood flow is reduced during the normal course of passive and active distribution control, red blood cells and fibrinogen act to form red blood cell clusters or rouleaux which can cause undesirable stoppage of microvascular flow. Rouleaux require certain levels of fluid shear stress to cause their breakup. When the blood's fibrinogen is high, fluid shear stress required to break up the rouleaux or to restart microvascular stoppage is commensurately higher and sufficient energy may not be available from the normal proximal flow. These aggregate phenomena can aggravate or promote local tissue anoxia, cerebral, myocardial or other organ infarctions and/or deep vein thrombosis. Aggregating phenomena also can occur due to stasis in a blood vessel brought about by surgical procedures. The aggregating tendency of red cells is a manifestation of attraction between their surfaces which can also be detected as an increase in sliding friction, if the cells are forced to move past each other slowly. These are the conditions easily attained in flow in the microcirculatory vessels: arterioles, capillaries, the venules, especially in the ever dividing channels of the arteriolar circuit and ever converging channels of the venular circuit. Typical inner diameters of these vessels range from 200 μm to 8 μm, and probably most of the arterio-venous blood pressure drop occurs in arterioles and capillaries having diameters less than 50 μm. Accordingly, it would be highly desirable to provide a determination of a patient's apparent blood viscosity under conditions relevant to flow in his microcirculatory vessels which is accurate, reproducible and simple to operate so that a patient can be appropriately diagnosed and treated in order to minimize or avoid the physiological risks of cellular aggregation.

Many presently available techniques for measuring blood viscosity are really aimed at measuring the end-point of surface-induced blood coagulation, the gel point, whereas in the described invention complications of coagulation are intentionally avoided. For example, US—A—3,587,295 discloses a procedure for measuring the coagulation characteristics of blood by subjecting the blood to mechanical energy and measuring the intensity of the energy transmitted to the blood which then is correlated with the coagulation characteristics of the blood. US—A—3,053,078 also utilizes an indirect method whereby a rotatable means is inserted into the blood and rotated at a constant velocity and the resistance to rotation then is measured and correlated with the coagulation characteristics of the blood. U.S. Patent 3,911,728 discloses a process for measuring blood viscosity by placing a blood sample and a confined gas in a tube having a narrow cross-section and reciprocally moving the blood through the narrow cross-section. The gas pressure variations due to compression of the gas are measured and then correlated with viscosity. Other indirect means for measuring physical characteristics of blood are shown in US—A—3,918,908, 3,967,934, 4,187,462 and 4,202,204. Since the means for measuring blood viscosity as disclosed in the cited patents are indirect, errors are introduced which render the results far less reliable than could be obtained with a direct blood viscosity measurement.

When the viscosity of anticoagulated blood is determined by conventional capillary viscometers, cone-and-plate viscometers, or cylindrical viscometers, the flow rates or shear rates are usually so high that the sliding friction and aggregating effects are obliterated, and the viscosity of the blood determined under such conditions appears to be both Newtonian (independent of flow rate) and dependent only on volume percent red cells (hematocrit). Consequently, the clinician has often relied on hematocrit reading as a guide to probable blood viscosity level, unaware of the fact that macromolecular plasma concentrations, especially of fibrinogen, can greatly increase the level of apparent viscosity that will be relevant in microcirculating flows.

Summary of the invention

In accordance with this invention as defined in appended claim 1, the apparent viscosity of blood is measured under conditions analogous to slow flow in the microcirculatory vessels by means of a porous bed, having pore dimensions comparable to the inner diameters of the microcirculatory vessels, whereby the sliding frictional effects, divisions of the flow and recombination of flow found in the living microcirculatory vessels can be approximated, and at the same time the flow through the bed is limited to a rate such that, in combination with the small pore size, the average wall shear stress is $10^{-1}$ N/m² (1 dyn/cm²) or less.

The apparatus includes a hollow transparent column in fluid communication with a chamber containing a porous bed. The porosity can be created in a variety of ways, for example, by packing fine spherical beads into a column, by synthesis of macroreticular networks from reagents like divinyl benzene or by phase separation of polyolefins. All that is required is that the porosity be reasonably uniform throughout the bed, to minimize channeling, and that the pore diameters fall in the range of approximately 100 μm to 200 μm, preferably from 10 μm to 50 μm. Furthermore, reproducibility from bed to bed is obviously desirable.

In use, blood is withdrawn from a patient and injected into the apparatus of this invention so that it permeates the porous bed and fills at least a portion of the hollow column. The blood sample then is allowed to pass through the porous bed and the flow rate of the blood in the column is measured. The lower the flow rate, the more viscous the blood sample. The flow condition created by the apparatus of this invention yields a viscosity value by measurement of the flow rate through a porous bed by the application of Darcy's Law:

$$Q/A = (B_o/\mu)\ (\Delta P/L)$$

wherein
$Q$ = volumetric flow rate, $cm^3/sec$
$A$ = total area of bed normal to flow, $cm^2$ (so that $Q/A$ is the approach velocity to the bed, cm/sec)
$B_o$ = Darcy specific permeability, $cm^2$
$\mu$ = viscosity of liquid, in $N.s/m^2$ (poise) (1 poise = 1 dyn-sec/cm²)
$L$ = total length of bed in direction of flow, cm
$\Delta P$ = pressure difference across bed, $N/m^2$ (dyn/ cm²)

In the device of this invention, the pressure difference is generated by a column of blood of average height h cm above the outlet, so that:

$$\Delta P = \rho g \bar{h}$$

where
$p$ = density of liquid (blood), $g/cm^3$ (about 1,0)
$g$ = gravitational acceleration, 980 cm/sec²

As practiced, it is convenient to measure volumetric flow rate Q by timing the fall of the upper meniscus of blood from a height $h_o$ to a final height $h_f$.
Thus,

$$\bar{h} = (h_o + h_f)/2.$$

The meniscus moves downward in a capillary tube of about 1 mm inside diameter, thus of cross-sectional areas

$$a_{cap} \cong 0,01\ cm^2.$$

Thus

$$Q = (h_o - h_f)a_{cap}./\Delta t$$

where
$\Delta t$ = time interval, seconds, for meniscus to fall from $h_o$ to $h_f$, typically about 1 cm.

It is found that the specific permeability of the bed should be about $6 \times 10^{-8}\ cm^2$, corresponding to 6 Darcy.

[1 Darcy = $0,986923 \cdot 10^{-8}$ cm (1 (cm³) (centipoise) (cm⁻¹) (sec⁻¹) (atm⁻¹))].

The equivalent radius of a capillary tube $r_e$ having the same Q/A under the same pressure gradient $\Delta P/L$ is given by the relation

$$r_e{}^2 = 8B_o$$

For

$$B_o = 6 \times 10^{-8}\ cm^2,\ r_e = 7 \times 10^{-4}\ cm = 7\ \mu m$$

The equivalent shear stress $\tau_e$ in the equivalent capillary is defined as:

$$\tau_e = (\Delta P/L)\ (r_w/2)\ N/m^2\ (dyn/cm^2)$$

so

$$\tau_e = (\Delta P/L)\sqrt{2B_o}$$

and under the preferred conditions ($\bar{h}$ = 5, L = 3, assuming $r_w = 7 \times 10^{-4}$,) $\tau_e \approx 0,06\ N/m^2$ (0,6 dyn/cm²).

The equivalent wall shear *rate* for the flow of a Newtonian liquid through the equivalent capillary is calculated as:

$$\dot{\gamma}_e = \tau_e/\mu$$

For blood plasma, $\mu \cong 0,001\ N.S/m^2$ (0,01 poise) and thus $\dot{\gamma} \cong 60\ sec^{-1}$ for the above example, but for blood, at low flow rates, its non-Newtonian properties could easily result in an effective $\mu$ of 0,01 (0,10) or higher, corresponding to $\dot{\gamma}_e$ of about 6 sec⁻¹ or less.

As will be seen, it is particularly convenient to make the hollow column diameter 1/10th or less the diameter of the porous bed; for example, 1 mm column diameter with a 1 cm bed diameter. As a consequence of the difference in area ratios by a factor of 100 thereby resulting, the approach velocity to the porous bed, which is inconveniently low in value, is reflected in a flow velocity through the hollow column 100-fold greater, leading to meniscus movements measurable in the range of centimeters per minute.

It is not essential to use freshly drawn blood from a patient. Blood can be drawn into anticoagulant sample tubes and tested subsequently in the device of this invention. However, it is believed that greatest accuracy is achieved when blood is drawn directly from the patient's vein and immediately introduced into this device. The device is preferably pre-warmed into this device.

The device is preferably pre-warmed to body temperature, 37°C. One of the outstanding advantages is the speed with which a determination can be made. The end-point reading can easily be obtained within 180 seconds from venopuncture, before coagulation begins. The ability to carry out the test so rapidly means that problems of platelet aggregation, possible changes in red cell stiffness due to storage or lack of dextrose, and other potential artifacts can be avoided. Furthermore, the temperature of the blood will necessarily be close to that of the body, 37°C, and thus, the viscosity measured will correspond to usual physiological temperatures in the microvasculature.

Brief descriptions of the drawings

Fig. 1 is a side view of the apparatus of this invention including a vent cap.

Fig. 2 is a side view of an embodiment of this invention including a vacuum tube.

Fig. 3 is a partial cross-sectional view showing measurement of blood flow in accordance with this invention.

Description of specific embodiments.

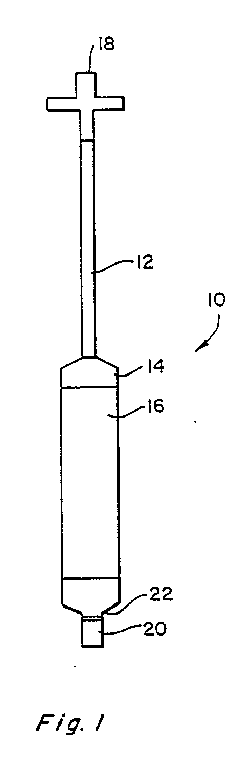

Referring to Fig. 1, the apparatus of this invention 10 includes a hollow column 12, and a chamber 14 which chamber contains a porous bed 16. A blood sample inlet 18 is provided at the top of the column 12 and a hydrophobic vent cap 20 is fitted over a blood outlet 22 at the lower end of chamber 14. A blood sample, preferably, but not necessarily taken immediately beforehand from the patient into a syringe by venipuncture, is introduced through inlet 18 and is allowed to flow through column 12 progressively filling chamber 14 while air is expelled through hydrophobic vent cap 20. The hydrophobic vent cap 20 prevents passage of blood therethrough so that, after the chamber 14 and column 12 are filled with blood, the vent cap 20 can be removed in order to initiate blood flow through the chamber 16 and the column 12.

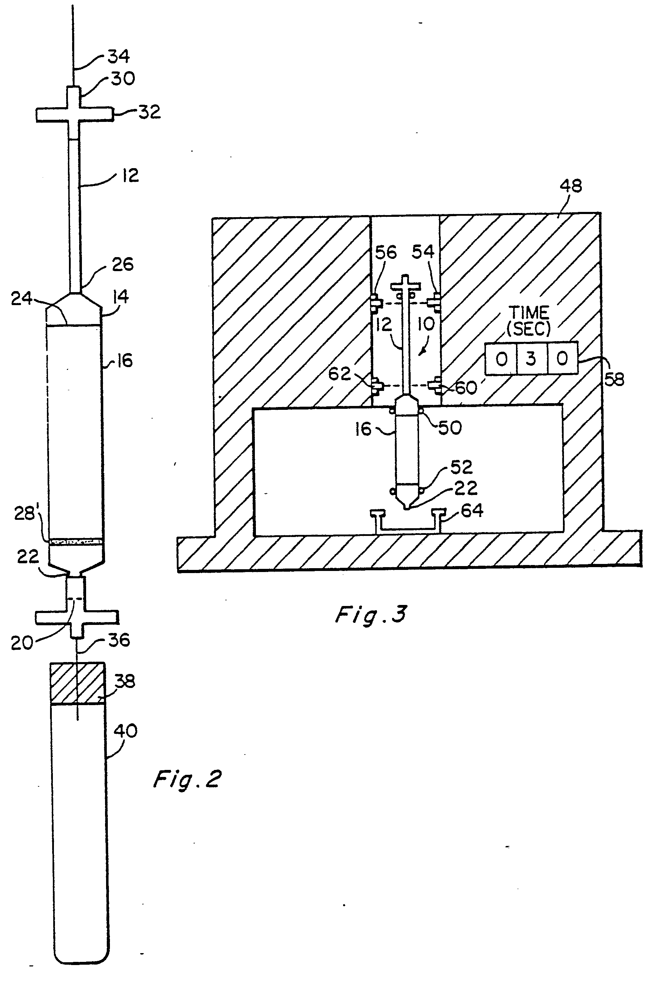

Referring to Fig. 2, an alternative embodiment is shown which includes means for taking a blood sample directly from the patient and introducing it into the apparatus of this invention. The apparatus includes a column 12, a chamber 14, and a porous bed 16. As for the device shown in Fig. 2, it is preferred that the top surface 24 of the porous bed 16 be spaced apart from the column outlet 26 in order to promote even flow across the horizontal cross-sectional areas of the packed bed 16. In addition, it is preferred that the packed bed 16 be spaced apart from the chamber outlet 22 such as by means of a screen 28 in order to promote even flow across the horizontal cross-section of the packed bed 16. Attached to the top of column 12 is a venipuncture device 30 which includes a holder 32 and a needle 34.

Venipuncture is accomplished in the usual way by grasping piece 32 so as to introduce needle 34 into the vein of a patient. Blood is introduced rapidly into the column 12 and the chamber 16 when needle 36 is inserted through seal 38 into tube 40 which is maintained under vacuum. The vacuum in tube 40 will cause blood eventually to flow through column 12, chamber 16 and into vacuum tube 40, unless hydrophobic vent cap 20 is interposed. In that case, the blood is stopped at that place.

Referring to Fig. 3, the blood viscometer 10 is placed within fixture 48 by means of clamps 50 and 52 after chamber 16 and column 12 have been filled with a patient's blood. Preferably, fixture 48 is thermostatically controlled at a constant temperature, preferably 37°C, in order to maintain the viscometer and blood contained therein at constant temperature. The hydrophobic vent cap (not shown) is removed from chamber outlet 22 so that blood flows by gravity down column 12, down chamber 16 so that the air-blood interfaces pass first between light emitting diode 54 and photodiode 56 which starts a conventional clock mechanism (not shown) having an associated time readout 58. As the air-blood interface passes downwardly through column 12, it passes a second set comprising a light emitting diode 60 and a photodiode 62 which causes the clock mechanism to stop. The operator then can easily read the elapsed time between the top set of diodes 54 and 56 and the bottom set of diodes 60 and 62 from the time readout 58. The blood passing through column 12 and chamber 16 flows into container 64. Since the blood flow rate through the column 12 depends upon its viscosity, the operator can easily determine the tendency of a particular patient to have abnormal microcirculation by comparing the time readout with a previously established standard.

The material utilized in the porous bed should not promote blood hemolysis and should allow blood permeation such that the average wall shear stress with blood is about $0,1$ $N/m^2$ (1 $dyn/cm^2$) or less. Suitable average pore sizes are between about 10 μm and about 200 μm, preferably between about 10 μm and about 50 μm. The bed should have a specific Darcy permeability of not more than 50 Darcy units ($50 \times 10^{-8}$ $cm^2$), preferably less than 10 Darcy units. Representative suitable particles include glass beads, preferably silane treated; polystyrene beads; polyethylene particles, and beds formed by sintering or related processes, for example, rods of sintered porous glass and analogous products produced by sintering granular plastic such as polyethylene, polypropylene, polyvinyl chloride, etc.

Generally, suitable bed thicknesses are between about 1 cm and about 10 cm, preferably between about 2 cm and about 4 cm and bed diameters are 0,4 to 2 cm, preferably 0,75 to 1,5 cm.

It is desirable that the apparatus of this invention does not require excessively large blood samples to be taken from the patient for testing. Therefore, the preferred bed sizes are set forth above while preferred column dimensions are of a height between about 1 cm and about 5 cm, preferably between about 2 cm and about 3 cm and an inside diameter between about 0,5 mm and about 2 mm, preferably around 1 mm and preferably not more than 1/10 the diameter of the porous bed.

It is obvious that the apparatus of Fig. 1 or of Fig. 2 could be initially filled with blood in the reverse direction to that shown, by placing hydrophobic vent cap 20 on opening 18 and introducing blood through opening 22.

In this case, readout apparatus of Fig. 3 would be modified by fixing clamps 50 and 52 on a frame having an axis of rotation slightly below the diode pair 60—62.

The frame through appropriate detent mechanism would have either of two vertical positions, differing by 180°: loading position (clamp 52 above 50, both above rotation axis), and reading position (clamp 52 under 50, both under axis of rotation) as shown in Fig. 3.

The apparatus after filling in the reverse direction is loaded into the clamps when they are in the loading position as described, thus with the chamber 14 above column 12, with hydrophobic vent 20 underneath.

The operator starts the readout by simply filling the frame to the reading position, which brings column 12 above chamber 14. Blood now drains from chamber 14 into receiver 64 as air is aspirated into column 12 through cap 20.

It is obvious that other means for measuring viscosity other than measuring blood flow rate directly can be utilized in the present invention. For example, the volume of blood in container 64 could be measured as a function of time and related to a previous standard that correlates volume with viscosity. Usually the devices will be pre-calibrated with standard fluids, for example physiologic saline solution and it will be known that, for example, the elapsed time between diodes is 6 seconds. If a sample of blood is found to take 30 seconds, its viscosity is then 30/6 or 5 times the viscosity of saline. Obviously, the standard fluid will be run in the device at the same temperature as the blood sample, preferably at or near 37°C, as explained above, for most patients.

The device can also be run at lower temperatures, for example 20°C, especially when cryoglobulinemia is suspected in the patient. If cryoglobulinemia is present, apparent blood viscosity will be drastically increased when measured at 20°C as compared to 37°C. In such a case, it would be desirable to take sufficient blood from the patient to fill two devices, and run one at 37°C and the other at 20°C.

## Claims

1. Apparatus for measuring blood viscosity which comprises a hollow column (12) in fluid communication with a chamber (14) containing a porous bed (16), means (18) for passing a blood sample through said hollow column (12) and said chamber (14) and means (54—64) for measuring the flow rate of blood through said chamber (14), the permeability ($B_o$) of said bed (16) and the pressure gradient across said bed ($\Delta P/L$) being such that the average wall shear stress defined as $(\Delta P/L) \sqrt{2B_o}$ is about $10^{-1}$ N/m$^2$ (1 dyn/cm) or less, and wherein the parameter "L" corresponds to the total length of the bed in the direction of flow and the pressure gradient is generated by the column of blood building up in the bed when blood passes through the bed by gravity.

2. The apparatus of claim 1 which includes a means (20) attached to its outlet (22) which permits passage of air therethrough while preventing passage of liquid therethrough.

3. The apparatus of any one of claims 1 or 2, including a venipuncture device (30) attached to its inlet and adapted to directly withdraw a blood sample from a patient into said apparatus (10).

4. The apparatus of any one of claims 1 or 2 including needle means (36) attached to its outlet (22) and adapted for being inserted into a vacuum thereby to permit withdrawing of blood sample through said apparatus (10).

5. The apparatus of claim 1 wherein said means for measuring the flow rate of blood through said chamber comprises optical means (54, 56; 60, 62) adapted to sense movement of an air-blood interface in said column (12).

6. The apparatus of claim 1 wherein the means for measuring the flow rate of blood through said chamber comprises a container (64) positioned at its outlet (22) adapted to provide a measurement of collected blood volume as a function of time.

## Patentansprüche

1. Vorrichtung zum Messen der Blutviskosität, mit einer hohlen Säule (12), die in Fluidverbindung mit einer ein poröses Bett (16) enthaltenden Kammer (14) steht, einer Einrichtung (18) zum Durchlassen einer Blutprobe durch die hohle Säule (12) und die Kammer (14) und einer Einrichtung (54—64) zum Messen der Fließgeschwindigkeit des durch die Kammer (14) fließenden Bluts, wobei die Permeabilität ($B_o$) des Bettes (16) und der Druckgradient längs des Bettes ($\Delta P/L$) derart gewählt sind, daß die als $(\Delta P/L) \sqrt{2.B_o}$ definierte, mittlere Wandscherbeanspruchung etwa $10^{-1}$ N/$^2$ (1 dyn/cm) oder weniger beträgt, und wobei der Parameter "L" der Gesamtlänge des Bettes in Richtung des Flusses entspricht und der Druckgradient durch eine Blutsäule erzeugt wird, die sich in dem Bett aufbaut, wenn das Blut durch das Bett infolge der Schwerkraft hindurchströmt.

2. Vorrichtung nach Anspruch 1, die eine an ihrem Auslaß (22) befestigte Einrichtung (20) aufweist, durch die Luft hindurchströmen kann, während das Durchströmen von Flüssigkeit verhindert wird.

3. Vorrichtung nach einem der Ansprüche 1 oder 2, die eine an ihrem Einlaß befestigte Venenpunktionseinrichtung (30) aufweist, die so ausgestaltet ist, daß eine Blutprobe von einem Patienten in die Vorrichtung (10) direkt zurückgezogen werden kann.

4. Vorrichtung nach einem der Ansprüche 1 oder 2, die eine an ihrem Auslaß (22) befestigte Nadeleinrichtung (36) aufweist, die in ein Vakuum eingesetzt werden kann, wodurch das Zurückziehen der Blutprobe durch die Vorrichtung (10) möglich ist.

5. Vorrichtung nach Anspruch 1, wobei die Einrichtung zum Messen der Fließgeschwindigkeit des durch die Kammer fließenden Bluts eine optische Einrichtung (54, 56; 60, 62) aufweist, die die Bewegung der Luft-Blut-Grenzfläche in der Säule (12) mißt.

6. Vorrichtung nach Anspruch 1, wobei die Einrichtung zum Messen der Fließgeschwindigkeit des durch die Kammer fließenden Bluts einen Behälter (64) aufweist, der an ihrem Auslaß (22) in Stellung gebracht ist, um eine Messung des gesammelten Blutvolumens als Funktion der Zeit vorzusehen.

**Revendications**

1. Appareil pour mesurer la viscosité du sang qui comprend une colonne creuse (12) en communication fluide avec une chambre (14) contenant un lit poreux (16), un moyen (18) pour faire passer un échantillon de sang à travers cette colonne creuse (12) et cette chambre (14), et des moyens (54—64) pour mesurer la vitesse d'écoulement du sang à travers cette chambre (14), la perméabilité ($B_o$) de ce lit (16) et le gradient de pression à travers ce lit ($\Delta P/L$) étant tels que l'effort de cisaillement moyen de la paroi, défini par ($\Delta P/L$) $\sqrt{2B_o}$ soit d'environ $10^{-1}$ N/m² (1 dyn/cm²) ou moins, et où le paramètre "L" correspond à la longueur totale du lit dans la direction de l'écoulement et le gradient de pression est généré par une colonne de sang s'établissant dans le lit quand le sang passe à travers le lit sous l'effet de la gravité.

2. Appareil selon la revendication 1, qui comprend un moyen (20) fixé à sa sortie (22) qui permet le passage de l'air tout en empêchant au liquide de passer.

3. Appareil selon l'une quelconque des revendications 1 ou 2, comprenant le dispositif de piqure intraveineuse (30) attaché à son entrée et conçu pour prélever directement un échantillon de sang du patient dans cet appareil (10).

4. Appareil selon l'une quelconque des revendication 1 ou 2, comprenant un moyen à aiguilles (36) fixé à sa sortie (22), et conçu pour être inséré dans un vide pour permettre le prélèvement d'échantillon de sang à travers cet appareil (10).

5. Appareil selon la revendication 1, dans lequel le moyen pour mesurer la vitesse d'écoulement du sang à travers cette chambre comprend des moyens optiques (54, 56; 60, 62) conçus pour détecter un mouvement de l'interface air-sang dans cette colonne (12).

6. Appareil selon la revendication 1, dans lequel le moyen pour mesurer la vitesse d'écoulement du sang à travers cette chambre comprend un récipient (64) disposé à sa sortie (22), conçu pour fournir une mesure du volume de sang collecté en fonction du temps.

*Fig. 1*

Fig.2

Fig.3

2